# EUROPEAN PATENT APPLICATION

(11) **EP 1 818 161 A1**
(43) Date of publication of application: **15.08.2007**
(21) Application number: 06002731.5
(22) Date of filing: 10.02.2006
(51) Int. Cl.: B29C 61/00, C08L 101/00, A61L 27/50

(54) **Shape memory polymers and shape memory polymer compositions responsive towards two different stimuli**

(71) Applicant: Mnemoscience GmbH, 52531 Uebach-Palenberg (DE)
(72) Inventor: Lendlein, Andreas, Dr., 14167 Berlin (DE)
(74) Representative: Grünecker, Kinkeldey, Stockmair & Schwanhäusser Anwaltssozietät

(57) **Abstract**

The present invention concerns shape memory polymers and shape memory polymer composition sensitive towards two different external stimuli.

## Description

The present invention concerns shape memory polymers and shape memory polymer compositions responsive to two different stimuli, such as light and temperature, temperature and pH value, and temperature and ion concentration, methods of producing same and the use of these materials.

### Prior art

Shape memory polymers are an interesting class of materials, which have received considerable attention in recent years. Shape memory functionality is the ability of a material to temporarily fix a second shape after an elastic deformation and only recover its original shape after application of an external stimulus. While this effect is one-way, reversible shape changes induced by cooling and heating, i.e. a two-way effect may also be realized.

Such a phenomenon is based on a structural phase transformation within the material. The advantageous and intriguing properties of these materials are in particular the possibility to initiate a desired change in shape by an appropriate external stimulus, so that an original shape, after deformation, is re-established, and the possibility to deform and program these materials so that highly specific configurations and shape changes can be obtained. The deformed shape is often called the temporary shape in the art. The phenomenon is a functionality and not an inherent material property. The effect/functionality results from a combination of polymer structure and specific functionalization processes.

The first materials known to provide this functionality were shape memory metal alloys. In the recent past, shape memory polymers have been developed. Typical shape memory polymers are, for example, phase segregated linear block copolymers, having a hard segment and a switching (soft) segment. Important representatives of these types of materials are disclosed in the international publications WO 99/42147 and WO 99/42528. These materials employ as external stimulus for initiating the recovery of the original shape a change in temperature, usually a temperature rise. Shape memory polymers being susceptible to other external stimuli are also known, such as the photosensitive shape memory polymers disclosed in WO 2004/062706.

The known shape memory materials may have one or more shapes in memory, illustrative examples being shape memory polymers having two shapes in memory, wherein each shape change may be triggered by a change in temperature. For certain applications, however, it is not suitable to employ a temperature rise for the triggering of both shape changes, as it is for example often difficult to provide shape memory materials of this kind, i.e. having two shapes in memory, with the required accuracy of distinction between the two triggering temperatures. Accordingly it is a desire in the art to provide shape memory materials, e.g. polymers and/or compositions, having more then one shape in memory, which overcome the above mentioned drawback.

Accordingly it is required to develop novel shape memory polymers and/or shape memory compositions having two shapes in memory.

### Brief description of the invention

The present invention solves the above object with the shape memory polymer and the shape memory polymer composition as defined in claim 1. Preferred embodiments are outlined in claims 2 to 7. Furthermore the present invention provides the method for preparing such shape memory polymers and shape memory polymer compositions as outlined in claims 8 and 9 as well as the use as defined in claim 10.

### Detailed description of the present invention

As defined in claim 1, the shape memory polymers and shape memory polymer compositions of the present invention comprise at least two different switching segments, so that the polymers and compositions of the present invention can memorize at least two shapes. As defined in the present invention these at least two different switching segments are sensitive towards different stimuli. Examples of stimuli which may be employed in accordance with the present invention are :
- change in temperature (Tsens)
- irradiation with light (Lsens)
- irradiation with gamma irradiation (Gsens)
- change in pH value (pHsens)
- change in ion concentration (Isens)
- treatment with ultrasound (Usens)
- subjection to magnetic fields (Msens)
- treatment wit water (Wsens)

The switching segments to be employed in accordance with the present invention are susceptible to such stimuli, the requirement for the shape memory polymers and shape memory polymer compositions of the present invention being that at least two different switching segments are present, sensitive towards at least two different stimuli. Suitable examples of combinations are as follows:
- (Tsens) / (Lsens)
- (Tsens) / (pHsens)
- (Tsens) / (Isens)
- (Tsens) / (Gsens)
- (Tsens) / (Usens)

Due to the selection of the switching stimuli in accordance with the requirement of the present invention it is possible to provide shape memory polymer and shape memory polymer compositions which are able to have at least two shapes in memory, wherein the at least two shape changes which may be triggered, are independent of one another. Independence in connection with the triggering of the shape memory effect, i.e. the desired initiation of a shape change, in accordance with the present invention comprises the following important features of the present invention. First it has to be emphasized that the selection of the switching segments/switching stimuli in accordance with the present invention enables that the at least two shape changes can be initiated with the desired accuracy, i.e. the desired first shape change may be initiated without risk that also the second shape change is triggered inadvertedly, as it may well be the case for two switching segments both being sensitive towards a change in temperature, wherein the two switching temperatures are not sufficiently distinct. The present invention enables that the desired accuracy of the triggering of the desired shape change is given, since the triggering of a shape change by a change in temperature (Tsens switching segment) will not affect a switching segment being sensitive towards light irradiation (Lsens switching segment). This is one important advantage associated with the present invention.

A further unique feature of the present invention is that the shape changes associated with the at least two switching segments, which are as outlined above sensitive towards different stimuli, may be triggered in any desired order. As outlined above, the triggering of one shape change by using the appropriate external stimulus for the respective switching segment, does not affect the at least one further switching segment. Accordingly the order of employing the external stimuli may be selected as required, since the shape memory effect may be initiated for each of the at least two different switching segments independent from the other. This is not possible with conventional shape memory polymers having two shapes in m emory, wherein both shape changes may be initiated by a temperature change. Such a material would have two transition temperatures, one being higher than the other. This however implies a certain order of programming and, importantly, of recalling the memorized shape. Such a restriction is not given for the materials in accordance with the present invention. The materials in accordance with the present invention may be programmed in a desired order, and likewise the shape memory effects may be initiated in any given, i.e. desired order, which does not need to be the order of programming. Accordingly the shape changes may be employed independent from the order of programming, enabling a greater degree of adaptation during use to the needs of the given field of application.

These advantages demonstrate the surprising properties of the shape memory polymers and shape memory polymer compositions of the present invention.

Concerning suitable molecular architectures for the materials of the present invention reference is made to the international publications mentioned above, which are incorporated herein. Important representatives of temperature sensitive segments are disclosed in the international publications WO 99/42147 and WO 99/42528. These materials may advantageously be used as temperature sensitive switching segments in the present invention. The preferred embodiments as derivable from these documents also apply for the present invention. Shape memory polymers being photosensitive are disclosed in WO 2004/062706. These materials may likewise be used in the present invention as light sensitive switching segments.

As outlined in these publications a suitable thermal transition for initiating the shape memory effect is a melting point (Tm) but also a glass transition point (Tg). Suitable segments as switching segments being sensitive towards a change in pH and/or ionic strength are polyelectrolyte segments.

Polyelectrolyte segments in accordance with the present invention are segments comprising a vast number of ionic groups, which may either be elements of the main chain of the segment or which may be elements of side chains of the main chain of the polyelectrolyte segment.

A polyelectrolyte segment in accordance with the present invention furthermore refers to a segment having a molecular weight of up to 15000, preferably 400 to 1 5000, more preferably 500 to 15000. Suitable embodiments of the molecular weight are also the ranges of from 1000 to 10000 and from 2500 to 7500.

The polyelectrolyte segments to be employed in accordance with the present invention can be distinguished as already indicated above very generally into segments wherein the ionic groups are comprised within the main chain (for example ionene) or they may be provided within side chains, such as in quarternized poly(4-vinylpyridin). The polyelectrolyte segments to be employed in accordance with the present invention furthermore can be classified broadly into polyacidic segments or polybasic segments. Polyacidic segments give rise to polyanions, while polybasic segments are segments comprising groups able to react with proton providing substances under the formation of salts. Typical examples of polyacidic polyelectrolyte segments are segments derived from polyphosphoric acid, segments derived from polyvinyl sulfuric acid, segments derived from polyvinyl sulfonic acid, segments derived from polyvinyl phosphonic acid and segments derived from polyacrylic acid. These groups can be derivatized further in any suitable manner. Other examples are alginate derived segments, i.e. segments derived from a Iginic acid. One advantage of these segments is the fact that alginates have long been used as thickeners or as components of pharmaceutical preparations, such as capsules, so that these materials are readily available from commercial sources. Furthermore there exists already knowledge concerning the processing of such materials.

Typical segments derived from polybasic polyelectrolytes are segments derived from polyethylene amine, polyvinyl amine and polyvinyl pyridine.

A third class of polyelectrolyte segments are ampholytic segments, comprising anionic as well as cationic groups, i.e. segments which give rise to polyions in suitable polar solvents.

All these types of polyelectrolyte segments may be employed in accordance with the present invention. Further polyelectrolytes which may be used as building blocks for segments of shape memory materials in accordance with the present invention may be selected from conventional polymers derivatized with groups providing anionic or cationic groups and conventional polyelectrolytes, such as polyallyl ammonium chloride, polyallyl ammonium phosphate, polydiallyldimethyl ammonium chloride, polybenzyltrimethyl ammonium chloride, polydiallyldimethyl ammonium chloride-co-N-isopropyl acryl amide, polysodiumtrimethylene oxyethylene sulfonate, polydimethyldodecyl-2-acrylamidoethylammonium bromide, poly-4-N-butylpyridinium methylene bromide, poly-2-N-methylpyridiniumethylene iodine, poly-N-methylpyridinium-2-5-diethylene, poly-4-4'-bipyridiniumdiayl-N,N'-decamethylenedibromide and betaine-derived polymers.

Important, in accordance with the present invention, is the fact that the polyelectrolyte segments to be employed in accordance with the present invention comprise groups enabling an ionic interaction between polymer segments.

The present invention uses the polyelectrolyte segments as switching segments, i.e. as soft segments. In this embodiment, the possibility to increase and to decrease the ionic interaction between different segments of a shape memory material in a reversible manner by means of a suitable manipulation is used in order to fix the temporary shape of a shape memory material. Such a shape memory material comprises either conventional network points or hard segments necessary for the memory concerning the permanent shape, or this material also employs polyelectrolyte derived segments as replacement for conventional hard segments or covalent network points, as outlined above. The temporary shape is then fixed by chemical manipulation leading to strong ionic interaction between polyelectrolyte segments in the deformed state. A recovery of the permanent shape can be triggered by appropriately changing the chemical composition with respect to the polyelectrolyte segments, for example, by providing additional reagents leading to a change in pH value or to salt exchange reactions. ln this connection, it is, for example, possible to replace a bridging divalent or trivalent cation, responsible for ionic interaction between anionic polyelectrolyte segments, by monovalent cations so that the bridging or crosslinking of different polyelectrolyte segments ceases to be present. This generates more freedom of movement of the segments by liberating the polyelectrolyte segments from one another so that a recovery of the original, permanent shape is made possible.

In one embodiment of using the polyelectrolyte segments as soft (switching) segments same are anionic segments initially present in association with monovalent, i.e. non-bridging cations. After a suitable deformation to the temporary shape the monovalent cations are exchanged and replaced by multivalent cations, preferably di- or trivalent cations, so that strong ionic interactions between the polyelectrolyte segments fix the temporary shape. The shape memory effect, i.e. the recovery of the permanent shape may then be initiated by replacing the bridging cations again with monovalent cations so that the interactions between the polyelectrolyte segments are reduced and finally extinguished so that the polymer recovers the permanent shape.

Another possibility is the initiation of the shape memory effect by altering the pH value, again with the aim of reducing and finally extinguishing interactions, which fix the temporary shape, between the polyelectrolyte segments.

The use of polyelectrolyte segments as soft segments in shape memory polymers widens considerably the range for suiable applications. In particular soft segments derived from polyelectrolyte segments enable the use of novel external stimuli for triggering the shape memory effect. Previously mainly temperature and light sensitive shape memory polymers have been reported. The novel materials in accordance with the present invention enable the use of other stimuli, such as ionic strength, pH value, type of ion (monovalent/multivalent cations, see above) etc. Such external stimuli further open new types of application, since the shape memory polymers in accordance with the present invention may be used in moist/liquid environments, since initiation of the shape memory effect requires the possibility to carry out ion exchange etc. which mainly may be realized in liquid systems. Accordingly the materials have to enable at least a certain degree of swelling, for example, in order to allow such reactions.

pH sensitive switching segments are in particular segments wherein groups allowing interactions, such as hydrogen bonding, which provide the temporary network points for fixing the temporary shape associated with this segment, are present. The shape memory effect in this respect may be initiated by adjusting the pH value to a predefined value at which such interactions are prevented.

The switching (soft) segment, i.e. the segment responsible for fixing the temporary shape and for providing the trigger for initiating the shape memory effect, of the shape memory polymers and compositions of the present invention may also be selected from segments providing network points / crosslinks, which may be provided in a usual manner, which are sensitive towards gamma irradiation. In the context of this embodiment of the present invention the term "sensitive towards gamma irradiation" defines the phenomenon that gamma irradiation leads to a chemical or physical change of the network points / crosslinks of the switching segment so that the fixation of the temporary shape is loosened and finally extinguished, so that the shape memory polymer or the product formed therefrom recovers the original permanent shape. Accordingly the present invention requires that the switching segments are selected so that the chemical/physical nature of the network points / crosslinks may be changed so that the fixation of the temporary shape ceases to exist. This may comprise distinct reactions leading to the cleavage of defined chemical or physical bonds, but the present invention also contemplates to employ rather unspecific degradation reactions in order to secure the desired shape change.

In accordance with the present invention it is therefore one suitable option to provide network points / crosslinks in the switching segments which comprise substantial amounts of aliphatic units or units comprising olefinic double bonds as network points / crosslinks. Gamma irradtiation is able to give rise to degradation reactions within such structures, so that the desired loosening and extinguishing of the network points / crosslinks may be achieved. Another alternative are functional groups providing the network points / crosslinks for securing/fixing the temporary shape, wherein these functional groups do show a well defined bond cleavage reaction upon irradiation with gamma rays. Suitable examples are cyclic moieties serving as crosslinks, which show a ring opening reaction upon irradiation with gamma rays.

In the following three possible structures of gamma ray sensitive switching segments in shape memory polymers are illustrated as reference.

A polymer material may comprise hard segments providing network points for fixing the permanent shape. The material further comprises flexible segments which are not sensitive towards irradiation (X-ray or gamma rays). These segments comprise groups or segments which are able to provide further crosslinks/network points after a deformation so that the deformed, i.e. temporary shape is fixed. These crosslinks/network points may be of physical or chemical nature (ionic interactions, covalent bonds, crystallites etc.). These groups or segments are sensitive towards irradiation with X-rays o gamma rays so that thereafter the crosslinks/network points are no longer present so that the material recovers the permanent shape. Materials of this type may be thermoplastic materials as well as network materials, depending in particular from the nature of the network points fixing the permanent shape.

One further option, in particular to provide shape memory polymers in the form of IPN or semi IPN is illustrated. An elastic network may for example be provided comprising flexible elastic segments having network points fixing the permanent shape. A telechelic oligomer, sensitive towards irradiation with X-rays or gamma rays is introduced, suitably by a procedure involving swelling, and the elastic network comprising the oligomer is then deformed to the temporary shape. This shape is then fixed by an appropriate reaction leading to a crosslinking of the oligomer, so that the new network, provided by the oligomer stabilizes the temporary shape. The permanent shape can then be recovered by destroying the network formed by the oligomer by irradiation with X-rays or gamma rays, since the oligomer is sensitive towards this type of irradiation whereas the elastic network responsible for the permanent shape is not affected thereby.

A further option for providing shape memory polymers in accordance with the present invention is illustrated in the following. This embodiment resembles the embodiment explained above. The polymer material comprises network points for the definition of the permanent shape. Further elastic segments are provided which comprise, either as terminal groups or as side groups functional groups, which, after deformation to the temporary shape are able to show some kind of interaction giving rise to further network points/crosslinks fixing the temporary shape. Again these network points/crosslinks are sensitive towards irradiation so that the permanent shape may be recovered after an appropriate treatment with X-rays or gamma rays. The elastic segments and the network points defining the permanent shape are again not affected by irradiation. In this embodiment it is preferred when the groups give rise to covalent bonds forming the network points/crosslinks fixing the temporary shape (of the Gsens phase).

Similar considerations also apply with respect to segments being sensitive towards treatment with ultrasound. Exchanging the gamma ray sensitive segments with ultrasound sensitive segments allows the use of ultrasound for initiating the shape memory effect.

The embodiment using switching segments being sensitive towards a treatment with water is an embodiment related o temperature sensitive switching segments. In this embodiment the switching segment is an amorphous segment having a Tg of above the use/application temperature of the product formed therefrom. In this respect this embodiment is very similar to the embodiments disclosed above in connection with the temperature sensitive segments. However, for the water sensitive segments the shape memory effect is initiated by penetrating water molecules, which act as softener for the segment. Thereby Tg is reduced so that Tg, after water treatment lies below the use/application temperature so that (in principle a thermal transition) the shape memory effect is initiated.

As already indicated above, the present invention concerns shape memory polymers as well as shape memory polymer compositions. Shape memory polymers in accordance with the present invention comprise the at least two different switching segments as defined in the present invention within the polymer structure, e.g. either as distinct blocks within a block-copolymer structure or as blocks within a copolymer structure wherein the blocks (corresponding to the switching segments) are present either within the main chain structure and/or as pendant side groups (graft copolymer structure). As outlined above the present invention also contemplates shape memory polymer compositions comprising the at least two different switching segments, an embodiment which may for example be realized in the form of a mixture of two shape memory polymers, wherein each shape memory polymer comprises one type of switching segment, so that in total the required at least two different switching segments are present. Such compositions/mixtures may be present in any desired and suitable form, such as thermoplastic polymer mixtures (blends), thermoplastic polymer mixtures subjected to a cross-linking treatment so that a network material results (network), as well as any kind of interpenetrating networks, such as IPN, and semi-IPN, differing with respect to the degree and kind of cross-linking. The type of shape memory polymer composition may be selected in accordance with the specific requirements in the desired field of application. In this respect reference is made to the well known synthesis procedures for preparing (multi)block copolymers as well as network polymers and IPN and semi-IPN as in particular identified in the above outlined earlier applications WO 99/42147, WO 99/42528, and WO 2004/062706 incorporated herein by reference.

Thermoplastic materials in particular may be prepared in the form of multiblock copolymers comprising one type of hard segment and two different types of switching segments. Covalent network structures may be prepared in the form of a network providing chemical (e.g. covalent) crosslinks responsible for the permanent shape and again two different types of switching segments. IPN materials may be prepared from two interpenetrating covalent networks which each comprise at least one type of hard segment (which may be same or different) and at least one type of switching segment, which are different. Semi-IPN materials may be prepared from blends of thermoplastic materials, again each comprising at least one type of hard segment (which may be same or different) and at least one type of switching segment, which are different. Finally mixed-IPN materials may be prepared from blend of thermoplastic materials and covalent network materials, again each comprising at least one type of hard segment (which may be same or different) and at least one type of switching segment, which are different.

Such shape memory polymers and/or shape memory compositions may be programmed as follows (i.e. definition and shaping of the temporary shapes):

First the permanent shape is formed, typically by conventional forming processes as disclosed in the prior art. Then the material is deformed to a desired first temporary shape and this shape is then fixed in accordance with the triggering mechanism of the switching segment (e.g. cooling for Tsens segments, pH variation etc). After the fixation of the first temporary shape, the material is deformed further to the desired second temporary shape. This shape again is the fixed in accordance with the triggering mechanism of the switching segment concerned. Additional programming steps may e added when more than two types of switching segments a present.

Recovery of the permanent and, prior thereto, of the temporary shape for which the necessary external stimulus has not yet been applied, can be accomplished in usual manner, i.e. by subjecting the material/product to the required external stimulus.

The shape memory polymers in accordance with the present invention show a structure corresponding to the known conventional shape memory polymers, in particular the shape memory polymers in accordance with the present invention comprise hard segments responsible for the permanent shape, which comprise network points / crosslinks of any suitable nature for providing and fixing the permanent shape. In particular suitable are covalent network points / crosslinks, although also other network points are suitable. In this connection reference is made to the international applications WO 99/42147, WO 99/42528, and WO 2004/062706, which are incorporated herein by reference concerning the chemical structure of the hard segments and the network points / crosslinks fixing the permanent shape. In this respect reference is made in particular to Figure 1 of WO 2004/062706 and the corresponding discussion in the specification of this document, showing an illustration of a shape memory material comprising covalent network points for fixing the permanent shape.

The segment structure and length for the hard segments may be selected as appropriate and in this connection reference is again made to the three international applications mentioned above, i.e. WO 99/42147, WO 99/42528, and WO 2004/062706.

The shape memory polymers of the present invention may be prepared using standard polymer reactions, such as disclosed in the above discussed international patent applications, i.e. WO 99/42147, WO 99/42528, and WO 2004/062706. In particular suitable is a method involving the provision of suitably functionalised macromonomers, corresponding to the segments of the shape memory polymers providing, for examples in diol form, followed by a reaction connecting the macromonomers, for example a reaction with a diisocyanate. Suitable reaction conditions are disclosed in WO 99/42147, WO 99/42528, and WO 2004/062706, incorporated herein by reference. Shape memory polymer compositions in accordance with the present invention may be prepared likewise in accordance with known principles of polymer chemistry and processing, for example using mechanical blending processed and/or chemical modifications, of polymer mixtures, for example using cross-linking agents.

The shape memory polymers of the present invention may be thermoplastic or thermoset materials, depending in particular from the desired end use. Molecular weight of the shape memory polymers and the individual segments, as well as molecular weight distribution and chemical composition may be adjusted in accordance with the knowledge of the skilled person, again in particular depending from the desired end use. The shape memory polymers of the present invention may be further blended with additional components, such as polymers, fillers, additives as well as pharmaceutical and diagnostic agents. In particular for the application in the medicinal field the shape memory polymers of the present invention may be compounded with active principles, such as anti-inflammatory agents, growth factors etc., as well as diagnostic agents, in particular contrast agents.

The shape memory polymers of the present invention are in particular intended for use in the medical field, as polymeric drug delivery agents, tissue regeneration skeletons etc. It is also possible apply the materials of the present invention in sensors in various fields, as actuators in various applications, in particular in complex structures, for medical devices, in particular in the field of minimal invasive chirurgical processes, as moldings in cars, for mounting and/securing/holding parts of devices (computer, television, etc.).

## Claims

1. Shape memory polymer or shape memory polymer composition, comprising at least two types of switching segments being sensitive towards different external stimuli.

2. Shape memory polymer or shape memory polymer composition in accordance with claim 1, wherein the at least two types of switching segments are sensitive towards stimuli selected from the group of change in temperature, irradiation with light, irradiation with gamma ray, change in p H value, and change in ion concentration.

3. Shape memory polymer in accordance with claim 1, wherein the at least two types of switching segments are selected so that the shape memory polymer or the shape memory polymer composition is sensitive towards a change in temperature and irradiation with light, a change in temperature and a change of pH value, or a change in temperature and a change of ion concentration.

4. Shape memory polymer in accordance with any of claims 1 to 3, wherein the at least two types of switching segments are present in the form of distinct blocks within a block-copolymer structure.

5. Shape memory polymer in accordance with any of claims 1 to 3, wherein the at least two different types of switching segments are present in the form of distinct bocks within the main chain of the polymer and as side chains of the polymer.

6. Shape memory polymer composition according to any of claims 1 to 3, wherein the composition forms a blend, a network structure, an IPN or a semi-IPN.

7. Method of programming a shape memory polymer or shape memory polymer composition according to any of claims 1 to 6, comprising the steps of:
a) shaping a shape memory polymer or shape memory polymer composition according to any of claims 1 to 6 into the desired permanent shape;
b) deforming the product obtained after step a) into a desired first temporary shape;
c) fixing the first temporary shape in accordance with the triggering mechanism of the switching segment for the first temporary shape;
d) deforming the product of step c) into a desired second temporary shape;
e) fixing the second temporary shape in accordance with the triggering mechanism of the switching segment for the second temporary shape.

8. Method of preparing a shape memory polymer in accordance with any of claims 1 to 5, comprising the reaction of suitably functionalized macromonomers corresponding to at least one type of the segments of the shape memory polymer with a suitable reactand for providing a chemical linkage between the segments.

9. Method of preparing a shape memory polymer composition in accordance with any of claims 6 or 7, comprising the step of providing at least two shape memory polymer components, blending the at least two different shape memory polymer components, and optionally subjecting the obtained blend to a post-blending reaction.

10. Use of a shape memory polymer or a shape memory polymer composition according to any of the preceding claims for preparing medical devices.
